(19) Europäisches Patentamt
European Patent Office
Office européen des brevets

(11) Numéro de publication : **0 594 484 A1**

(12) # DEMANDE DE BREVET EUROPEEN

(21) Numéro de dépôt : **93402542.0**

(22) Date de dépôt : **15.10.93**

(51) Int. Cl.[5] : **C07D 239/42,** C07D 239/46, C07D 401/12, A61K 31/505, A61K 31/535

(30) Priorité : **22.10.92 FR 9212642**

(43) Date de publication de la demande : **27.04.94 Bulletin 94/17**

(84) Etats contractants désignés : **AT BE CH DE DK ES FR GB GR IE IT LI LU MC NL PT SE**

(71) Demandeur : **SYNTHELABO
22, Avenue Galilée
F-92350 Le Plessis Robinson (FR)**

(72) Inventeur : **George, Pascal
19 rue des quatre Vents
F-78730 St Arnoult en Yvelines (FR)**
Inventeur : **Marabout, Benoit
6 rue Georges Ritz
F-91300 Massy (FR)**
Inventeur : **Merly, Jean-Pierre
11 Avenue Jules Guesde
F-92330 Sceaux (FR)**
Inventeur : **Froissant, Jacques
Cidex 981, Brevainville
F-41160 Morée (FR)**

(74) Mandataire : **Ludwig, Jacques et al
SYNTHELABO, Service Brevets, B.P. 72
F-92352 Le Plessis Robinson Cédex (FR)**

(54) **Dérivés de 2-aminopyrimidine-4-carboxamide, leur préparation et leur application en thérapeutique.**

(57) Composés répondant à la formule générale (I)

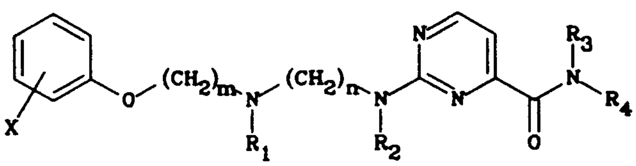

(I)

dans laquelle m et n valent chacun 2 ou 3, X représente H, F, Cl, $-OCH_3$, $-OC_2H_5$ ou $iC_3H_7$, $R_1$ et $R_2$ représentent chacun H ou $CH_3$, $R_3$ représente H, $R_4$ représente un groupe alkyle en $C_1$-$C_3$, un groupe $-CH_2CONH_2$, un groupe $-CH_2CON(CH_3)_2$, un groupe $-CH_2CH(OH)CH_2OCH_3$, un groupe $-CH_2CH(OCH_3)CH_2OCH_3$, un groupe $-(CH_2)_2$-Ar (où Ar est un phényle éventuellement substitué), un groupe (oxopyrimidinyl)amino($C_2$-$C_3$)alkyle, un groupe aroylamino($C_2$-$C_3$)alkyle, ou bien encore $R_3$ et $R_4$ forment, avec l'atome d'azote qui les porte, un groupe morpholin-1-yle ou pipérazin-1-yle éventuellement substitué en position 4.
Application en thérapeutique.

EP 0 594 484 A1

La présente invention a pour objet des dérivés de 2-aminopyrimidine-4-carboxamide, leur préparation et leur application en thérapeutique.

Les composés de l'invention répondent à la formule générale (I)

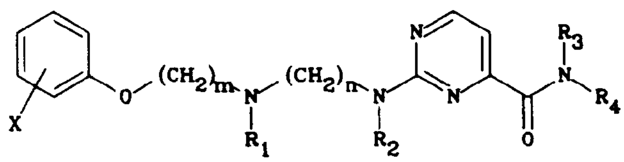

(I)

dans laquelle

m représente le nombre 2 ou 3,

n représente le nombre 2 ou 3,

X représente un ou plusieurs atomes ou groupes choisis parmi les suivants : hydrogène, 1-méthyléthyle, fluor, chlore, méthoxy, éthoxy,

$R_1$ représente un atome d'hydrogène ou un groupe méthyle,

$R_2$ représente un atome d'hydrogène ou un groupe méthyle,

$R_3$ représente un atome d'hydrogène ou un groupe alkyle en $C_1$-$C_3$,

$R_4$ représente

. soit un groupe alkyle en $C_1$-$C_3$,

. soit un groupe de formule générale $-CH_2-CO-N(R_5)_2$ (dans laquelle $R_5$ représente un atome d'hydrogène ou groupe méthyle),

. soit un groupe (hydroxy) (méthoxy)propyle ou diméthoxypropyle,

. soit un groupe de formule générale $-(CH_2)_2-Ar$ (dans laquelle Ar représente un groupe phényle éventuellement substitué),

. soit un groupe de formule générale $-(CH_2)_q-SO_2-R_6$ ou $-(CH_2)_q-NH-SO_2-R_5$ (dans lesquelles q représente le nombre 2 ou 3 et $R_5$ représente un groupe alkyle en $C_1$-$C_3$, un groupe aryle ou un groupe amino),

. soit un groupe de formule générale

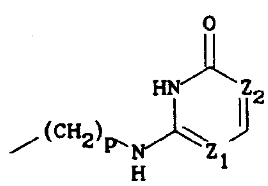

ou (tautomère)

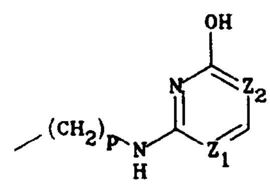

dans laquelle p représente le nombre 2 ou 3, l'un des symboles $Z_1$ et $Z_2$ représente un groupe CH et l'autre représente un atome d'azote,

. soit un groupe de formule générale

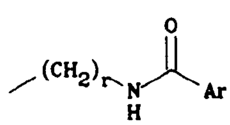

dans laquelle r représente le nombre 2 ou 3 et Ar représente un groupe phényle éventuellement substitué ou un groupe pyridin-3-yle,

ou bien encore

$R_3$ et $R_4$ forment, avec l'atome d'azote qui les porte, un groupe de formule générale

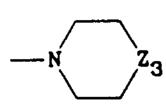

dans laquelle $Z_3$ représente un atome d'oxygène, un groupe de formule générale $N-R_7$ ou $CH-(CH_2)_s-NH-R_7$ (dans lesquelles s représente le nombre 0 ou 1 et $R_7$ représente un atome d'hydrogène, un groupe

méthyle ou un groupe de formule générale

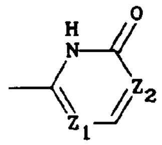

ou (tautomère)

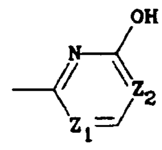

dans laquelle $Z_1$ et $Z_2$ sont tels que définis ci-dessus).

Les composés de l'invention peuvent se présenter à l'état de bases libres ou de sels d'addition à des acides pharmaceutiquement acceptables.

Conformément à l'invention, on peut préparer les composés de formule générale (I) selon un procédé illustré par le schéma qui suit.

On transforme un amide de formule générale (II), dans laquelle m, n, X, $R_1$ et $R_2$ sont tels que définis ci-dessus, en ester de formule générale (III) dans laquelle R' représente un groupe alkyle en $C_1$-$C_4$, par réaction avec un alcool aliphatique, par exemple le méthanol, en présence d'un acide, par exemple l'acide chlorhydrique gazeux, à une température de 0 à 60°C, puis on fait réagir l'ester ainsi obtenu avec une amine de formule générale $R_3$-NH-$R_4$, dans laquelle $R_3$ et $R_4$ sont tels que définis ci-dessus, dans un alcool aliphatique, par exemple le méthanol ou le n-butanol, à une température de 0 à 100°C.

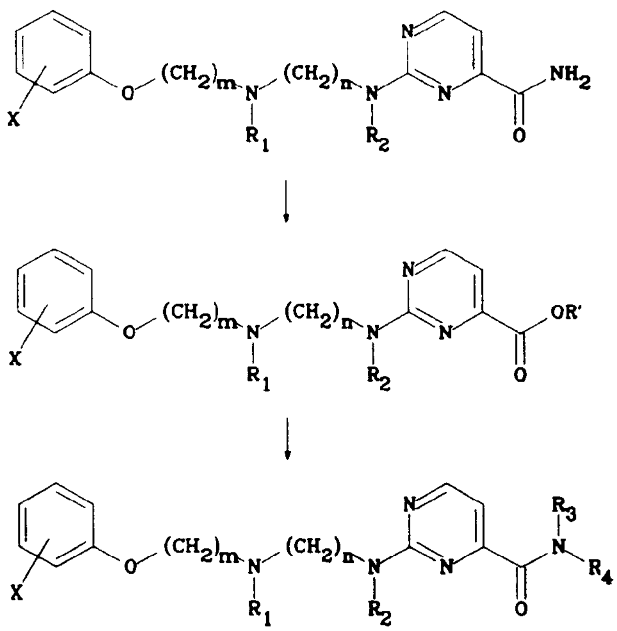

(II)

(III)

(I)

Les composés de départ de formule générale (II) peuvent être préparés par des méthodes analogues à celles décrites dans la demande de brevet EP-0511072.

Les amines de formule générale $R_3$-NH-$R_4$ dans laquelle $R_3$ représente un atome d'hydrogène et $R_4$ représente un groupe de formule générale

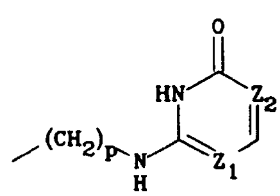

peuvent être préparées par des méthodes analogues à celles décrites dans *Biochem. Biophys. Res. Comm.* **170**(1), 243 (1990) et dans la demande de brevet EP-0373891.

Les amines de formule générale $R_3$-NH-$R_4$ dans laquelle $R_3$ représente un atome d'hydrogène et $R_4$ représente un groupe de formule générale

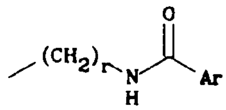

peuvent être préparées par des méthodes analogues aux méthodes mentionnées ci-dessus.

Enfin dans le cas des amines de formules générale $R_3$-NH-$R_4$ dans laquelle $R_3$ et $R_4$ représentent ensemble un groupe de formule

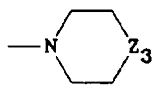

dans laquelle $Z_3$ représente un groupe N-$R_7$ ou CH-$(CH_2)_n$-NH-$R_7$ tel que défini ci-dessus, on utilise une telle diamine protégée, dans la formule de laquelle $R_7$ représente un groupe protecteur tel qu'un groupe benzyloxycarbonyle ou tert.-butyloxycarbonyle, que l'on peut préparer par des méthodes analogues à celles décrites dans les demandes de brevets DE-2831431, EP-0410278 et EP-0417698.

Si on le désire, on déprotège le composé obtenu selon une méthode connue, par exemple par l'acide trifluoroacétique dans le dichlorométhane (dans le cas d'un groupement tert.-butyloxycarbonyle), pour obtenir un composé dans la formule duquel $R_7$ représente l'hydrogène et, si on le désire, on fait réagir cette dernière avec la 2-méthylthiopyrimidin-4(1*H*)-one, selon une méthode analogue à celle décrite dans la demande de brevet EP-0373891.

Il va de soi que les diverses modifications qui viennent d'être décrites à propos de l'amine protégée de formule générale $R_3$-NH-$R_4$ peuvent être effectuées directement sur ladite amine, comme décrit ci-dessus, mais aussi sur le composé de formule générale (I), après la réaction de l'amine protégée avec l'ester de formule générale (III).

Les exemples suivants illustrent la préparation de quelques composés selon l'invention.

Les micronalyses élémentaires et les spectres IR et RMN confirment les structures des produits obtenus.

Les numéros de composés indiqués entre parenthèses dans les titres des exemples correspondent à ceux du tableau donné plus loin.

Exemple 1 (Composé N°3)

Ethanedioate de *N*-(2-amino-2-oxoéthyl) -2-[[3-[(2-phénoxyéthyl)méthylamino]propyl]amino]pyrimidine-4-carboxamide (1:1).

1.1 2-[[3-[(2-Phénoxyéthyl)méthylamino]propyl]amino]pyrimidine-4-carboxylate de méthyle.

Dans un ballon de 1 l, on introduit 2,42 g (7,35 mmoles) de 2-[[3-[(2-phénoxyéthyl)méthylamino]propyl]amino]pyrimidine-4-carboxamide et 350 ml de méthanol, puis on fait passer un courant d'acide chlorhydrique gazeux pendant quelques minutes et on chauffe à la température du reflux du méthanol pendant 5 heures.

On évapore le solvant sous pression réduite, on ajoute au résidu 250 ml de dichlorométhane et on refroidit à 0°C. On alcalinise le mélange avec une solution aqueuse saturée hydrogénocarbonate de sodium, on décante et sèche la phase organique sur sulfate de sodium, on filtre puis on évapore le solvant sous pression réduite.

Après purification par chromatographie sur colonne de silice (éluant : mélange dichlorométhane/méthanol 100/0 à 85/15), on isole 2,28 g (6,62 mmoles) de composé sous forme huileuse qu'on utilise tel quel dans l'étape suivante.

1.2 Ethanedioate de *N*-(2-amino-2-oxoéthyl)-2-[[3-[(2-phénoxyéthyl)méthylamino]propyl]amino]pyrimidine-4-carboxamide (1:1).

Dans un ballon de 100 ml on introduit 1,14 g (3,31 mmoles) de 2-[[3-[(2-phénoxyéthyl)méthylamino]propyl]amino]pyrimidine-4-carboxylate de méthyle, 1,4 g (12,66 mmoles) de chlorhydrate de glycinamide en solution dans 15 ml de méthanol, puis on ajoute 1,45 g (14,35 mmoles) de triéthylamine et on chauffe le mélange au reflux pendant 24 heures.

On refroidit à température ambiante, on évapore le solvant sous pression réduite et on ajoute au résidu brut 150 ml de dichlorométhane et 100 ml d'eau. On sépare la phase organique, on la sèche sur sulfate de sodium, on filtre et on évapore le solvant sous pression réduite.

On purifie le produit brut par chromatographie sur gel de silice (éluant : dichlorométhane/méthanol 92/2

à 80/20) pour obtenir 0,79 g de base.

On dissout la base dans 40 ml de méthanol et on ajoute 0,26 g (2 mmoles) d'acide oxalique. On réduit le volume des 2/3 puis on additionne du propan-2-ol pour obtenir 0,61 g (1,28 mmoles) de composé.

Point de fusion : 145-147°C.

Exemple 2 (Composé N°8)

Ethanedioate de *N*-[3-(benzoylamino)propyl]-2-[ [3-[ [2-(2-méthoxyphénoxy)éthyl]méthylamino]propyl]amino]pyrimidine-4-carboxamide.

2.1 2-[[3-[[2-(2-Méthoxyphénoxy)éthyl]méthylamino]propyl]amino]pyrimidine-4-carboxylate de méthyle.

Dans un ballon de 0,5 l, on introduit 12,7 g (35,3 mmoles) de 2-[[3-[[2-(2-méthoxyphénoxy)éthyl]méthylamino]propyl]amino] pyrimidine-4-carboxamide et 300 ml de méthanol puis on fait passer un courant d'acide chlorhydrique gazeux pendant quelques minutes et on chauffe à la température du reflux du méthanol pendant 4 heures.

On évapore le solvant sous pression réduite, on ajoute au résidu 100 ml de dichlorométhane et on refroidit à 0°C. On alcalinise le mélange avec une solution aqueuse saturée d'hydrogénocarbonate de sodium, on décante et sèche la phase organique sur sulfate de sodium, on filtre puis on évapore le solvant sous pression réduite.

Après purification par chromatographie sur colonne de silice (éluant : mélange dichlorométhane/méthanol, 96/4 à 88/12), on isole 8,7 g (23,24 mmoles) de composé 2.1 sous forme huileuse qu'on utilise tel quel dans l'étape suivante.

2.2 Ethanedioate de *N*-[3-(benzoylamino)propyl]-2-[[3-[[2-(2-méthoxyphénoxy)éthyl]méthylamino]propyl]amino]pyrimidine-4-carboxamide.

Dans un ballon de 100 ml, on introduit 1,2 g (3,2 mmoles) de 2-[[3-[[2-(2-méthoxyphénoxy)éthyl]méthylamino]propyl]amino] pyrimidine-4-carboxylate de méthyle et 1,06 g (5,95 mmoles) de *N*-(3-aminopropyl)benzamide en solution dans 10 ml d'un mélange 4/1 de propan-2-ol/méthanol, puis on chauffe le milieu réactionnel au reflux pendant 16 heures.

On refroidit à température ambiante, on évapore les solvants sous pression réduite puis on chromatographie l'huile obtenue sur gel de silice (éluant : dichlorométhane/méthanol, 97/3 à 90/10) pour obtenir 1,4 g de base.

On dissout la base dans 30 ml d'éthanol et on ajoute 0,24 g (2,7 mmoles) d'acide oxalique. On évapore sous pression réduite puis on recristallise le résidu solide dans un mélange propan-2-ol/acétate d'éthyle pour obtenir 1,3 g (2,13 mmoles) de composé.

Point de fusion : 138-140°C.

Exemple 3 (Composé N°11)

(*E*) But-2-ènedioate de 2-[[3-[[2-(2-méthoxyphénoxy)éthyl]méthylamino]propyl]amino]-*N*-[2-[(4-oxo-3,4-dihydropyrimidin-4-ylamino]éthyl]pyrimidine-4-carboxamide (1:1).

Dans un ballon de 100 ml, on introduit 2,3 g (6,14 mmoles) de 2-[[3-[[2-(2-méthoxyphénoxy)éthyl]méthylamino]propyl]amino]pyrimidine-4-carboxylate de méthyle et 0,95 g (6,14 mmoles) de 2-[(2-aminoéthyl)amino]pyrimidin-4(1*H*)-one dans 20 ml de n-butanol. On chauffe le mélange à la température du reflux du solvant pendant 31 heures, puis on évapore le solvant sous pression réduite.

On purifie le produit brut par chromatographie sur colonne de gel de silice (éluant : mélange dichlorométhane/méthanol, 90/20 à 70/30) pour obtenir 0,95 g de base.

On dissout la base dans 20 ml d'éthanol et on ajoute 0,22 g (1,87 mmoles) d'acide fumarique dans 20 ml d'éthanol. On réduit le volume des 9/10, on ajoute de l'acétate d'éthyle pour provoquer la cristallisation, et on obtient finalement 0,95 g (1,5 mmoles) de composé.

Point de fusion : 108-110°C.

Exemple 4 (Composé N° 17)

Dichlorhydrate de 1-[[2-[[3-[[2-(5-chloro-2-méthoxyphénoxy)éthyl]méthylamino]propyl]amino]pyrimidin-4-yl]carbonyl]-4-méthylpipérazine.

4.1 2-[[3-[[2-(5-chloro-2-méthoxyphénoxy)éthyl]méthylamino]propyl]amino]pyrimidine-4-carboxylate de méthyle.

Dans un ballon de 0,25 l, on introduit 4,8 g (12,2 mmoles) de 2-[[3-[[2-(5-chloro-2-méthoxyphénoxy)éthyl]méthylamino]propyl]amino]pyrimidine-4-carboxamide et 100 ml de méthanol puis on fait passer un courant d'acide chlorhydrique gazeux pendant quelques minutes et on chauffe à la température du reflux du méthanol pendant 2 heures. On évapore le solvant sous pression réduite, on ajoute au résidu 100 ml de dichlorométhane et on refroidit à 0°C. On alcalinise le mélange avec une solution aqueuse saturée d'hydrogénocarbonate de sodium, on décante et sèche la phase organique sur sulfate de sodium, on filtre puis on évapore le solvant sous pression réduite.

Après purification par chromatographie sur colonne de gel de silice (éluant : mélange dichlorométhane/méthanol, 98/2 à 90/10), on isole 3,3 g (8,07 mmoles) de composé sous forme huileuse, qu'on utilise tel quel dans l'étape suivante.

4.2 Dichlorhydrate de 1-[[2-[[3-[[2-(5-chloro-2-méthoxyphénoxy)éthyl]méthylamino]propyl]amino]pyrimidin-4-yl]carbonyl]-4-méthylpipérazine.

Dans un ballon tricol de 250 ml, on introduit successivement 25 ml de dichlorométhane, 7,6 ml d'une solution à 25 % de triméthylaluminium dans de l'hexane puis 1,8 g (18,1 mmoles) de 1-méthylpipérazine dans 15 ml de dichlorométhane. On agite le mélange réactionnel pendant 30 minutes à la température ambiante, on ajoute alors lentement 1,2 g (2,94 mmoles) de 2-[[3-[[2-(5-chloro-2-méthoxyphénoxy)éthyl]méthylamino]propyl]amino]pyrimidine-4-carboxylate de méthyle dans 20 ml de dichlorométhane. On agite le mélange réactionnel pendant 30 minutes à la température ambiante puis pendant 2,5 heures à la température du reflux.

On refroidit le mélange réactionnel à 0°C avec un mélange eau/sel/glace, on hydrolyse par quelques ml d'eau, on laisse agiter 1 heure à la température ambiante, on filtre, on ajoute de l'eau et on extrait à l'acétate d'éthyle. On lave la phase organique à l'eau, on la sèche sur sulfate de sodium, on filtre puis on évapore le solvant sous pression réduite. On purifie l'huile obtenue par chromatographie sur colonne de gel de silice (éluant :: dichlorométhane/méthanol, 50/100 à 85/15) pour obtenir 1,35 g de base.

A la base en solution dans 10 ml de dichlorométhane, on ajoute 53 ml d'acide chlorhydrique 0,1N dans du propan-2-ol, on évapore sous pression réduite et on fait cristalliser le résidu dans un mélange propan-2-ol/acétate d'éthyle. On obtient 1,2 g (2,18 mmoles) de dichlorhydrate.

Point de fusion : 116-119°C.

Le tableau qui suit illustre les structures chimiques et les propriétés physiques de quelques composés selon l'invention.

## Tableau

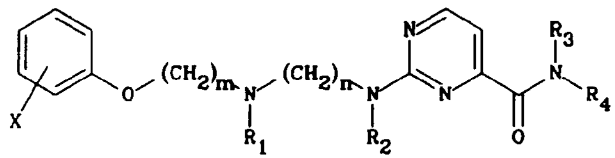

(I)

| N° | X | m | $R_1$ | n | $R_2$ | $-NR_3R_4$ | Sel | F (°C) |
|---|---|---|---|---|---|---|---|---|
| 1 | 4-F | 2 | $CH_3$ | 3 | H | $-NH-CH_3$ | 2HCl | 144,5-145,5 |
| 2 | 4-F | 2 | $CH_3$ | 3 | H | $-NH-(CH_2)_2-SO_2-CH_3$ | Ox. | 152-153,5 |
| 3 | H | 2 | $CH_3$ | 3 | H | $-NH-CH_2-CO-NH_2$ | Ox. | 145-147 |
| 4 | 4-F | 2 | $CH_3$ | 3 | H | $-NH-CH_2-CO-N(CH_3)_2$ | Ox. | 165-168 |
| 5 | 2-$OCH_3$ | 2 | $CH_3$ | 3 | H | $-NH-CH_2-CH(OCH_3)-CH_2-OCH_3$ | Ox. | 65-68 |
| 6 | 4-F | 2 | $CH_3$ | 3 | H | $-NH-CH_2-CH(OH)-CH_2-OCH_3$ | Ox. | 69-72 |
| 7 | 4-F | 2 | $CH_3$ | 3 | H | $-NH-(CH_2)_2-NH-CO-$ 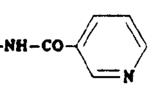 | - | 94,5-95,5 |

| N° | X | m | $R_1$ | n | $R_2$ | $-NR_3R_4$ | Sel | F (°C) |
|---|---|---|---|---|---|---|---|---|
| 8 | $2-OCH_3$ | 2 | $CH_3$ | 3 | H | 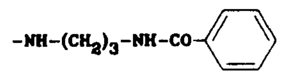 | Ox. | 138-140 |
| 9 | $2-OCH_3$ | 2 | $CH_3$ | 3 | H | 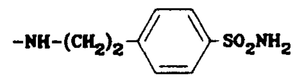 | 2HCl | 105-108 |
| 10 | $2-OCH_3$, 5-Cl | 2 | $CH_3$ | 3 | H | 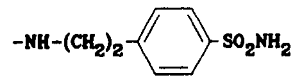 | - | 67-70 |
| 11 | $2-OCH_3$ | 2 | $CH_3$ | 3 | H | 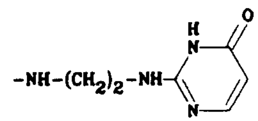 | Fum, $2H_2O$ | 108-110 |
| 12 | $2-OC_2H_5$ | 2 | $CH_3$ | 3 | H | 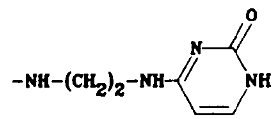 | - | 167-169 |
| 13 | $2-OCH_3$, 5-F | 2 | $CH_3$ | 3 | H | 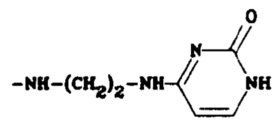 | - | 173-176 |
| 14 | $2-OCH_3$ | 2 | $CH_3$ | 3 | H | 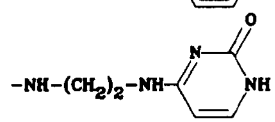 | - | 152-154 |
| 15 | H | 2 | $CH_3$ | 3 | H | 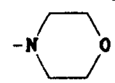 | 2HCl | 171-174 |
| 16 | $2-OCH_3$ | 2 | $CH_3$ | 3 | H | 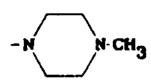 | 2HCl, $2H_2O$ | 102-105 |

| N° | X | m | $R_1$ | n | $R_2$ | $-NR_3R_4$ | Sel | F (°C) |
|---|---|---|---|---|---|---|---|---|
| 17 | $2\text{-}OCH_3$, 5-Cl | 2 | $CH_3$ | 3 | H | 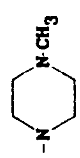 | 2HCl,$2H_2O$ | 116-119 |
| 18 | $2\text{-}CH(CH_3)_2$ | 2 | $CH_3$ | 3 | H | 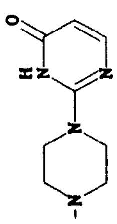 | - | 55-59 |

Légende

Dans la colonne "Sel", "-" désigne un composé à l'état de base, "Ox." désigne un oxalate (éthanedioate) acide, "Fum" désigne un fumarate ((*E*) but-2-ènedioate) acide, "2HCl" désigne un dichlorhydrate et "$2H_2O$" désigne un dihydrate.

Les composés de l'invention ont fait l'objet d'études quant à leur activité antagoniste des récepteurs al-adrénergiques au niveau du bas appareil urinaire.

Leur activité *in vitro* a été étudiée sur l'urètre isolé de lapin.

On prépare des anneaux d'urètre de lapin mâle adulte selon la méthode de Ueda et al., *Eur. J. Pharmacol.*, (1984), **103**, 249-254, puis, après sensibilisation à la noradrénaline, on détermine la courbe concentration-réponse à la phényléphrine, en absence et en présence de composé à étudier.
On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.
Les $pA_2$ des composés sont de l'ordre de 5,5 à 9,0.

L'activité *in vivo* des composés de l'invention a été étudiée quant à leur effet sur l'hypertonie urétrale engendrée par la stimulation des fibres sympathiques du nerf hypogastrique chez le chat anesthésié.
On anesthésie des chats mâles adultes au pentobarbital sodique, et on les prépare selon la méthode de Theobald, *J. Auton. Pharmac.*, (1983), **3**, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.
On évalue la puissance de l'antagonisme $\alpha_1$-adrénergique de chaque composé par calcul de la $DI_{50}$, dose qui inhibe de 50% l'hypertonie urétrale.
Les $DI_{50}$ des composés de l'invention sont de l'ordre de 0,01 à 1 mg/kg.

Les résultats des essais montrent que les composés de l'invention montrent, *in vitro,* une activité antagoniste des récepteurs $\alpha_1$-adrénergiques des muscles lisses du bas appareil urinaire (urètre) stimulés par un agoniste $\alpha_1$-adrénergique (phényléphrine). *In vivo,* ils inhibent l'hypertonie urétrale engendrée par la stimulation nerveuse sympathique.

Les composés de l'invention peuvent donc être utilisés pour le traitement symptomatique des maladies et affections impliquant une hyperactivité du système $\alpha$-adrénergique au niveau du bas appareil urinaire, et notamment pour le traitement des troubles mictionnels de l'hypertrophie bénigne de la prostate, tels que la dysurie et la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,1 à 500 mg de substance active.

## Revendications

1. Composé répondant à la formule générale (I)

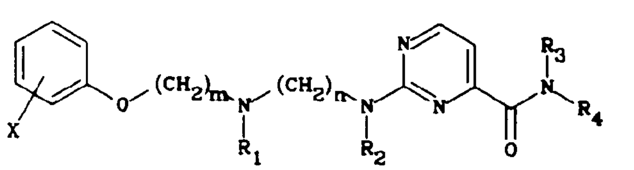

(I)

dans laquelle
m représente le nombre 2 ou 3,
n représente le nombre 2 ou 3,
X représente un ou plusieurs atomes ou groupes choisis parmi les suivants : hydrogène, 1-méthyléthyle, fluor, chlore, méthoxy, éthoxy,
$R_1$ représente un atome d'hydrogène ou un groupe méthyle,
$R_2$ représente un atome d'hydrogène ou un groupe méthyle,
$R_3$ représente un atome d'hydrogène,
$R_4$ représente
. soit un groupe alkyle en $C_1$-$C_3$,
. soit un groupe de formule générale $-CH_2-CO-N(R_5)_2$ (dans laquelle $R_5$ représente un atome d'hydrogène ou groupe méthyle),
. soit un groupe (hydroxy)(méthoxy)propyle ou diméthoxypropyle,
. soit un groupe de formule générale $-(CH_2)_2-Ar$ (dans laquelle Ar représente un groupe phényle éventuellement substitué),
. soit un groupe de formule générale $-(CH_2)_q-SO_2-R_6$ ou $-(CH_2)_q-NH-SO_2-R_6$ (dans lesquelles q repré-

sente le nombre 2 ou 3 et $R_6$ représente un groupe alkyle en $C_1$-$C_3$, un groupe aryle ou un groupe amino),

. soit un groupe de formule générale

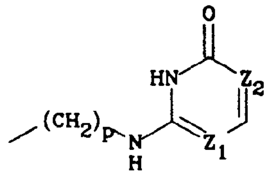

ou (tautomère)

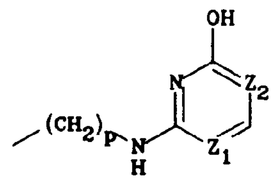

dans laquelle p représente le nombre 2 ou 3, l'un des symboles $Z_1$ et $Z_2$ représente un groupe CH et l'autre représente un atome d'azote,

. soit un groupe de formule générale

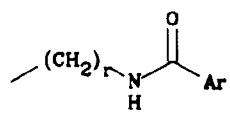

dans laquelle r représente le nombre 2 ou 3 et Ar représente un groupe phényle éventuellement substitué ou un groupe pyridin-3-yle,

ou bien encore

$R_3$ et $R_4$ forment, avec l'atome d'azote qui les porte, un groupe de formule générale

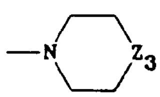

dans laquelle $Z_3$ représente un atome d'oxygène, un groupe de formule générale N-$R_7$ ou CH-$(CH_2)_s$-NH-$R_7$ (dans lesquelles s représente le nombre 0 ou 1 et $R_7$ représente un atome d'hydrogène, un groupe méthyle ou un groupe de formule générale

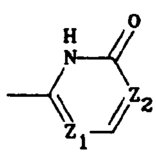

ou (tautomère)

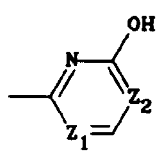

dans laquelle $Z_1$ et $Z_2$ sont tels que définis ci-dessus), à l'état de base libre ou de sel d'addition à un acide.

**2.** Procédé de préparation d'un composé selon la revendication 1, caractérisé en ce qu'on transforme un amide de formule générale (II)

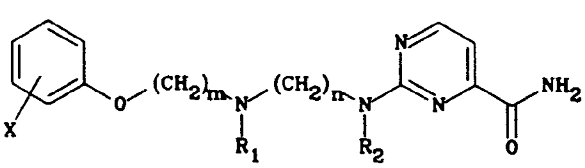

(II)

dans laquelle m, n, X, $R_1$ et $R_2$ sont tels que définis dans la revendication 1, en ester de formule générale (III)

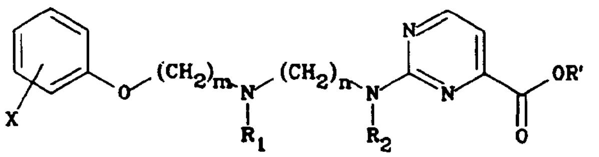

(III)

dans laquelle R' représente un groupe alkyle en $C_1$-$C_4$, par réaction avec un alcool aliphatique, en présence d'un acide, à une température de 0 à 60°C, puis on fait réagir l'ester ainsi obtenu avec une amine de formule générale $R_3$-NH-$R_4$, dans laquelle $R_3$ et $R_4$ sont tels que définis dans la revendication 1, dans un alcool aliphatique, à une température de 0 à 100°C.

3. Médicament caractérisé en ce qu'il consiste en un composé selon la revendication 1.

4. Composition pharmaceutique caractérisée en ce qu'elle contient un composé selon la revendication 1, associé à un excipient pharmaceutique.

Office européen des brevets

# RAPPORT DE RECHERCHE EUROPEENNE

Numero de la demande
EP 93 40 2542

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 480 794 (SYNTHELABO)<br>* le document en entier *<br>--- | 1-4 | C07D239/42<br>C07D239/46<br>C07D401/12<br>A61K31/505<br>A61K31/535 |
| P,A | EP-A-0 511 072 (SYNTHELABO) 28 Octobre 1992<br>* le document en entier *<br>----- | 1-4 | |
| | | | DOMAINES TECHNIQUES RECHERCHES (Int.Cl.5) |
| | | | C07D |

Le présent rapport a été établi pour toutes les revendications

| Lieu de la recherche | Date d'achèvement de la recherche | Examinateur |
|---|---|---|
| LA HAYE | 6 Janvier 1994 | Francois, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)